# EUROPEAN PATENT SPECIFICATION

(11) **EP 3 320 881 B1**
(45) Date of publication and mention of the grant of the patent: **04.09.2019**
(21) Application number: 17275167.9
(22) Date of filing: 20.10.2017
(51) Int. Cl.: A61F 2/07, A61F 2/95

(54) **DIAMETER REDUCTION CONSTRAINT ARRANGEMENT FOR A STENT GRAFT IN COMBINATION WITH A STENT GRAFT**
DURCHMESSERVERKLEINERUNGSEINSCHRÄNKUNGSANORDNUNG FÜR EIN STENTIMPLANTAT IN KOMBINATION MIT EINEM STENTIMPLANTAT
AGENCEMENT DE CONTRAINTE DE RÉDUCTION DE DIAMÈTRE EN COMBINAISON AVEC UNE PROTHÈSE VASCULAIRE MUNIE DE STENTS

(30) Priority: 10.11.2016 AU 2016256777; 10.11.2016 US 201615347907
(43) Date of publication of application: 16.05.2018
(73) Proprietor: Cook Medical Technologies LLC, Bloomington, IN 47404 (US)
(72) Inventor: KING, Chantelle, Woodridge, Queensland 4114 (AU); SMITH, Logan, Mount Gravatt, Queensland 4122 (AU)
(74) Representative: Williams Powell

(56) References cited:
- EP-A1- 2 471 498
- EP-A1- 3 040 056
- US-A- 5 405 378
- US-A1- 2014 148 895

## Description

### Technical Field

This invention relates to medical devices and more particularly to stent grafts mountable onto a deployment device for endovascular introduction.

### Background Art

This invention will be particularly discussed in relation to stent grafts for placement into the thoracic abdominal aorta or into the abdominal aorta for the treatment of aneurysms. The invention, however, is not so restricted and may be applied to stent grafts for placement in any lumen of the human or animal body.

The positioning of stent grafts is very important for a number of reasons, including in many cases the need to avoid occlusion of branch arteries. Positioning is complicated however because the diameter of a stent graft is deliberately made larger than the diameter into which it is to be placed to allow for accurate sealing against the vessel wall, possible errors in sizing and subsequent relaxation of the vessel wall.

Once released from a delivery device, a stent graft with self-expanding stents will take up a position against the vessel wall and it will be difficult if not impossible to reposition it. This can be dangerous if, for instance, renal arteries are occluded.

Stent graft assemblies with diameter reducing ties have been developed to assist surgeons with the task of positioning stent grafts after initial release from a deployment device. Sewing and positioning of such ties during manufacture can be difficult and time consuming. Furthermore, errors in this process can have adverse effects.

US 5405378 describes a device comprising a prosthesis designed as a hollow body compressed against the action of restoring spring forces to a cross section reduced relative to an expanded use position, and held in this position by a strippable sheath. After the sheath is stripped, the prosthesis automatically expands to a cross section corresponding to the use position. The sheath, which can be a meshwork in the approximate form of crocheted material, extends over the entire length of the prosthesis and consists of at least one continuous thread and at least one drawstring. The prosthesis, held in the radially compressed position by the sheath, can be mounted displaceably on a feed wire or non-axially-displaceably on the insertion end of a probe or a catheter.

### Summary of the Invention

The present invention seeks to address difficulties described above while at the same time improving safety and simplifying assembly or to at least provide a useful alternative assembly technique.

According to an aspect of the present invention, there is provided a temporary diameter reduction constraint arrangement for a stent graft in combination with a stent graft, the stent graft having a proximal end and a distal end and comprising a biocompatible graft material tube and a plurality of longitudinally spaced apart self-expanding stents fastened thereto, including at least an end stent and a plurality of intermediate stents, the constraint arrangement comprising:
an elongate receiver extending longitudinally within the graft material tube;
a first wire extending longitudinally along the graft material tube in a first serpentine pattern; and
a second wire extending longitudinally along the graft material tube in a second serpentine pattern,
wherein at least one of the first and second wires repeatedly loops over the receiver, within the graft material, along a longitudinal length of the stent graft thereby securing the stent graft to the receiver.

Advantageously, the first and second serpentine patterns cross each other in a criss-cross lacing pattern.

Preferably, the first and second wires repeatedly cross each other within the graft material tube.

In preferred embodiments, the first and second wires lie predominately inside the graft material tube.

Advantageously, the first and second wires repeatedly penetrate a tubular wall of the graft material tube from inside the graft material tube to outside the graft material tube and then penetrate the tubular wall back from outside the graft material tube to inside the graft material tube, thereby forming a plurality of external wire portions.

Each intermediate stent may be a zig-zag stent comprising struts and bends forming peaks and valleys and wherein the intermediate stents are longitudinally spaced apart such that their respective peaks are substantially longitudinally aligned.

In preferred embodiments, the external wire portions are disposed within a V, or an inverted V, formed between adjacent struts of one of the plurality of intermediate stents.

The receiver may be a temporary loading member, provided to facilitate loading of the constraint arrangement onto a guide wire cannula. The temporary loading member may comprise a tube having a through-bore.

The receiver may be a guide wire cannula.

The constraint arrangement may include a plurality of loops of thread, each loop engaged with one or other of the first and second wires and engaged around a portion of the stent graft circumferentially spaced a selected distance away from its wire, and drawn and tied to itself to reduce the stent graft.

A detailed description of one or more embodiments of the invention is provided below along with accompanying figures that illustrate by way of example the principles of the invention. For the purpose of example, numerous specific details are set forth in the following description in order to provide a thorough understanding of the teachings herein.

Throughout this specification the term distal with respect to a portion of the aorta, a deployment device or a prosthesis is the end of the aorta, deployment device or prosthesis further away in the direction of blood flow away from the heart and the term proximal means the portion of the aorta, deployment device or end of the prosthesis nearer to the heart. When applied to other vessels similar terms such as caudal and cranial should be understood.

For the purpose of clarity, technical material that is known in the related technical fields has not been described in detail so that the teachings herein are not unnecessarily obscured.

### Brief Description of the Drawings

Embodiments of the present invention are described below, by way of example only, with reference to the accompanying drawings, in which:
Figure 1 shows a stent graft according to the prior art;
Figure 2 is a perspective view of a temporary diameter reduction constraint arrangement for a stent graft in combination with a stent graft according to a first embodiment of the invention;
Figure 3 is a similar view to that of Figure 2, but shows the constraint arrangement in a partially reduced configuration;
Figure 4 is a perspective end view of the constraint arrangements shown in Figures 2 and 3 looking in from a proximal end thereof;
Figure 5 is a similar view to that of Figure 4, over a guide wire cannula of a delivery device;
Figures 6A to 6C show an embodiment of the invention as part of a delivery device;
Figure 7 shows an embodiment similar to that shown in Figure 3 but having diameter reducing ties or sutures;
Figures 8A and 8B are detailed views showing the sutures or ties of Figure 7 in an initial expanded condition and in a constrained or reduced condition respectively;
Figure 9 is an isometric side view showing the constraint arrangement of Figure 7 in a side view;
Figure 10 shows an embodiment of the invention as part of a delivery device; and
Figure 11 shows another embodiment of the invention in an isometric view.

### Description of Preferred Embodiments

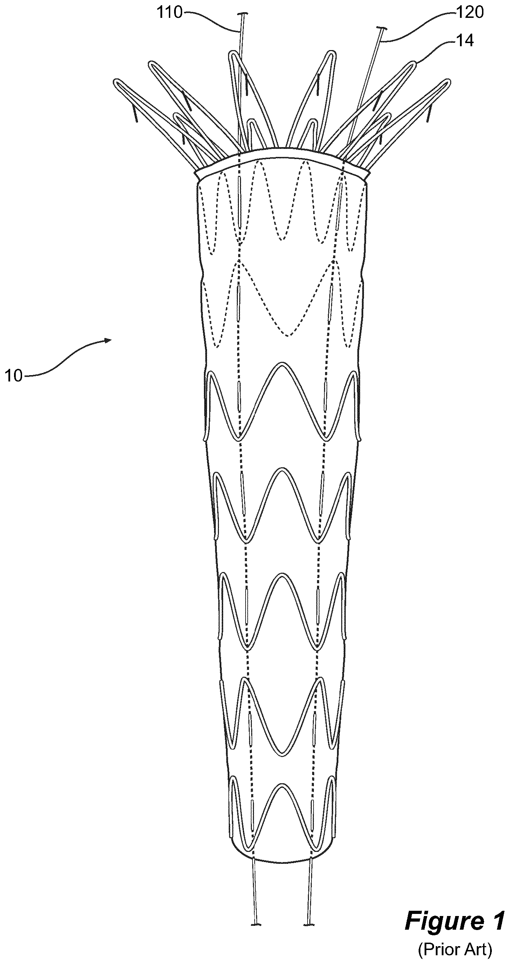

Referring to Figure 1, a stent graft of the type shown in US Patent Application Serial Number US-2007/0043425, entitled "Assembly of Stent Grafts" is shown. This stent graft 10 has release wires 110 and 120 that can be used together with reducing ties to achieve a reduction in the circumference of the stent graft 10 as is explained in the specification of US-2007/0043425.

A temporary diameter reduction constraint arrangement for a stent graft in combination with a stent graft is shown and described in US Patent Application Serial Number US-2014/0148895, entitled "Assembly of stent grafts with diameter reducing ties".

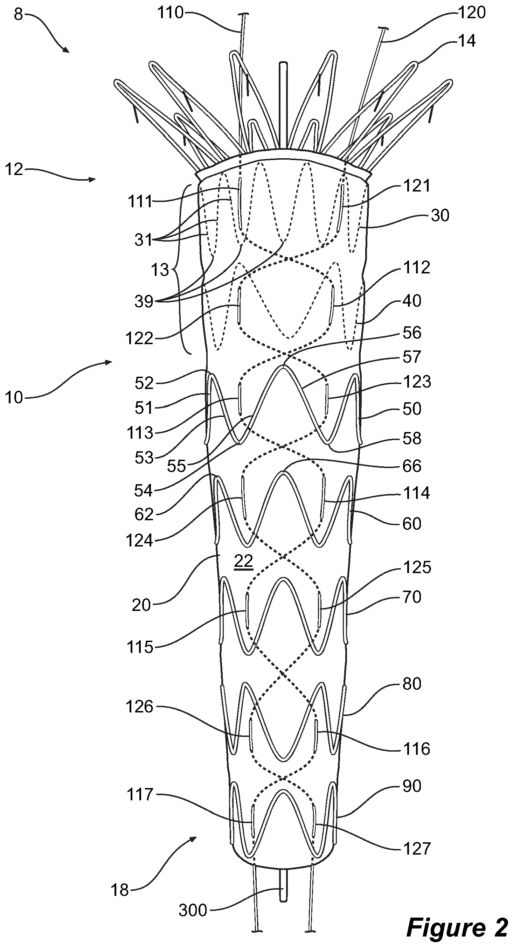

Referring to Figure 2, a temporary diameter reduction constraint arrangement 8 for a stent graft 10 in combination with a stent graft 10 according to a first embodiment of the invention is shown. The stent graft 10 has a proximal end 12 and a distal end 18 and comprises a biocompatible graft material tube 20 of a selected diameter. It also has a plurality of longitudinally spaced apart self-expanding stents fastened thereto, including at least an end stent and a plurality of intermediate stents. More specifically, the embodiment's stent graft has two internal stents 30, 40 shown in dotted lines and a plurality of external stents 50, 60, 70, 80, 90 along the length of its tubular body, the stents 40, 50, 60, 70 and 80 being intermediate stents and the stents 30 and 90 being end stents. The internal stents 30, 40 are at the proximal end 12 and act on a sealing zone 13 also at the proximal end 12.

Preferably, the graft tube material 20 may be formed from a biocompatible material that is substantially non-toxic in the in vivo environment of its intended use and substantially not rejected by the patient's physiological system (i.e., is non-antigenic). For example, the graft tube material 20 may be made of an expanded polytetrafluoroethylene (ePTFE), polytetrafluoroethylene, silicone, polyurethane, polyamide (nylon), polyethylene, polypropylene, polyaramids, polyacrylonitrile, cellulose, or another flexible biocompatible material. The graft tube material 20 also may be made of known fabric graft materials, e.g., woven polyester such as DACRON.RTM. from Invista (Wichita, Kans.), polyetherurethanes such as THORALON.RTM. from Thoratec Corporation (Pleasanton, Calif.), or polyethylene such as an ultra-high molecular weight polyethylene (UHMwPE) such as DYNEEMA.RTM. from DSM Dyneema LLC (Stanley, N.C.). In addition, materials that are not inherently biocompatible may be subjected to surface modifications to render the materials biocompatible. Examples of surface modifications include, for example, graft polymerization of biocompatible polymers on the surface, coating of the surface with a crosslinked biocompatible polymer, chemical modification with biocompatible functional groups, or immobilization of a compatibilizing agent such as heparin or other biocompatible substances. Thus, any fibrous material having sufficient strength to survive in the in vivo environment may be used to form a textile graft, provided the final textile is biocompatible.

The graft tube material 20 material also may include a bioremodelable material such as reconstituted or naturally-derived collagenous materials. Suitable remodelable materials can be provided by collagenous extracellular matrix (ECM) materials possessing biotropic properties. For example, suitable collagenous materials may include ECM materials such as those comprising submucosa, renal capsule membrane, dermal collagen, dura mater, pericardium, fascia lata, serosa, peritoneum or basement membrane layers, including liver basement membrane. Suitable submucosa materials for these purposes may include, for instance, intestinal submucosa including small intestinal submucosa, stomach submucosa, urinary bladder submucosa, and uterine submucosa. Collagenous matrices including submucosa (potentially along with other associated tissues) useful for this purpose can be obtained by harvesting such tissue sources and delaminating the submucosa-containing matrix from smooth muscle layers, mucosal layers, and/or other layers occurring in the tissue source. For additional information as to some of the materials useful in the present invention, and their isolation and treatment, reference can be made, for example, to US Patent Numbers 4,902,508, 5,554,389, 5,993,844, 6,206,931, and 6,099,567. Non-limiting example of suitable remodelable materials may include SURGISIS.RTM. BIODESIGN.TM. from Cook Medical (Bloomington, Ind.) or the graft prosthesis material described in US Patent Number 6,206,931 to Cook et al. The graft tube material also may be made of any of the materials described in US Patent Number 7,407,509 to Greenberg et al. or US Patent Application Publication Number 2009/0171451 to Kuppurathanam et al.

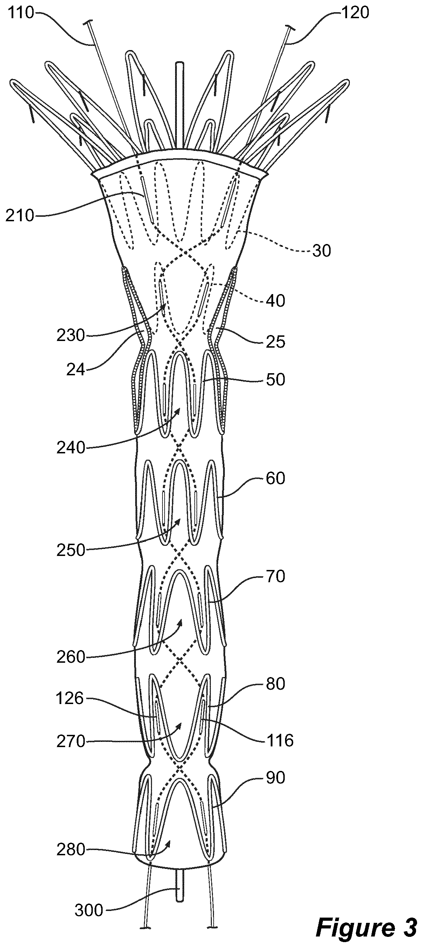

Figure 3 is a similar view to that of Figure 2, but shows the constraint arrangement in a reduced condition in which the diameter is reduced. This view also shows optional fenestrations 24 and 25 which can be provided for allowing access to the renal arteries. Embodiments of the invention can facilitate matching such fenestrations 24 and 25 up with the renal arteries when the stent graft is deployed into an aorta. Methods of deployment of such a stent graft are described in PCT Patent Publication Number WO98/53761 entitled "A Prosthesis and a Method of Deploying a Prosthesis". These features and other features disclosed in PCT Patent Publication Number WO98/53761 could be used with the present invention.

Although the stent graft 10 shown is useable in aortas in the region of the renal arteries, the invention may be embodied in other stent grafts, which may or may not have fenestrations.

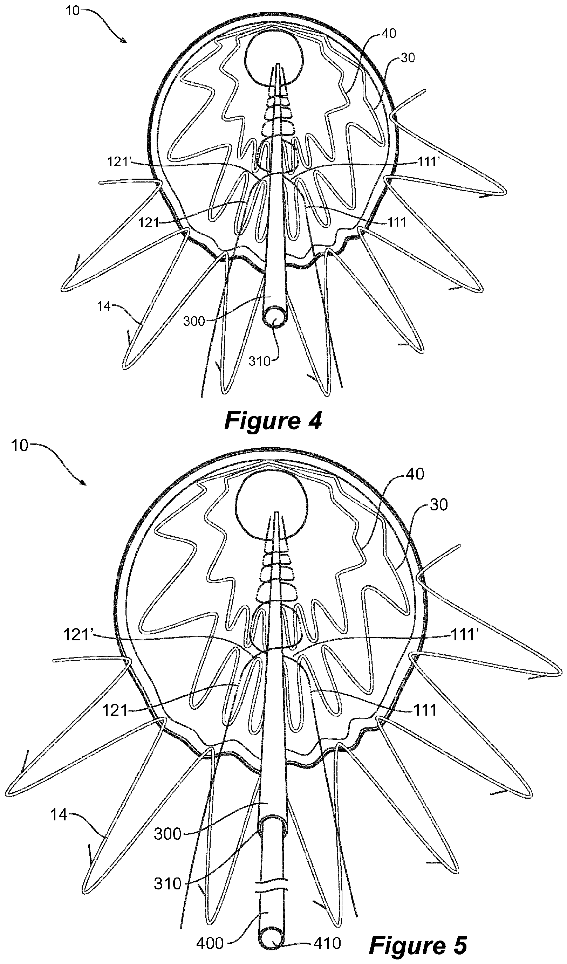

The constraint arrangement shown in Figures 2 and 3 comprises an elongated receiver 300 extending longitudinal within the graft material tube 20. This elongated receiver 300 is more clearly shown in Figure 4. In this embodiment, the elongate receiver 300 is a tube with a through-bore in the form of an internal bore 310.

The constraint arrangement further comprises a first wire 110 extending longitudinal along the graft material tube 20 in a first serpentine pattern, and a second wire 120 extending longitudinally along the graft material tube 20 in a second serpentine pattern. At least one of the first and second wires 110,120 repeatedly loops over the receiver 300 along a longitudinal length of the stent graft 10 thereby securing the stent graft 10 to the receiver 300.

Referring again to Figures 2 and 4, it can be seen that the first and second serpentine patterns cross each other in a criss-cross lacing pattern. It can also be seen in Figure 4 that the first and second wires 110,120 repeatedly cross each other within the graft tube material 20. The first and second wires 110,120 lie predominantly inside the graft material tube 20.

As can be seen in Figures 4 and 5, the wires 110, 120 are arranged to sit on opposite sides of the receiver 300. This reduces wire to wire contact. Wire contact can be undesirable in some instances as it can potentially increase the chances of the creation on wire damage, shavings or entanglement.

The first and second wires 110,120 repeatedly penetrate a tubular wall 22 of the graft material tube 20 from inside the graft material tube 20 to outside the graft material tube 20 and then penetrate the tubular wall 22 back from outside the graft material tube 20 to inside the graft material tube, thereby forming a plurality of external wire portions including portions 124 and 114 shown in Figure 2. Wire 110, as a result of the repeated penetrations just described, creates external wire portions 111, 112, 113, 114, 115, 116 and 117. Similarly, wire 120 forms external wire portions 121, 122, 123, 124, 125, 126 and 127.

The stents of the embodiments of the invention may have any suitable stent pattern known in the art. The stents may be balloon expandable. Preferably, the stents may be self-expandable. The stents can maintain the patency of the prosthesis and ensure adequate sealing against the surrounding vascular tissue. One goal for stent design and placement, whether internal or external, may be to prevent metal-to-metal contact points, prevent contact between two different types of alloys, and minimize micromotion. Stent sizing, spacing, and design may be determined so that there is no stent-to-stent contact even in tortuous anatomy. Stents preferably may be placed to maximize prosthesis flexibility while maintaining patency, as well as reduce material wear and stent fatigue. Furthermore, it is preferable that the stents do not interfere with the branch, that they minimize the potential for galvanic corrosion, and ensure adequate joint stability. Stent amplitude, spacing, and stagger preferably may be optimized for each prosthesis design. Any of the stents mentioned herein may have barbs and/or other anchoring members to help decrease prosthesis migration.

One example of a stent pattern is the Z-stent or Gianturco stent design. Each Z-stent may include a series of substantially straight segments or struts interconnected by a series of bent segments or bends. The bent segments may include acute bends or apices. The Z-stents are arranged in a zigzag configuration in which the straight segments are set at angles relative to one another and are connected by the bent segments. This design provides both significant radial force as well as longitudinal support. In tortuous anatomy, branches, or fenestrations, it may be preferable to use other stents or modifications to the Z-stent design to avoid stent-to-stent contact. Other stents may include, for example, annular or helical stents. Furthermore, in complex anatomical situations, external stents may have the potential to become intertwined with the wires or other devices utilized to ensure branch vessel access, sealing, and fixation. Thus, in some instances, it may be desirable to affix some of the stents to the internal surface of the prosthesis.

The stents described herein may be made from any suitable material known in the art. In one example, the stents may be made from standard medical grade stainless steel and are soldered using silver standard solder (0 lead/0 tin). In other examples, the stents may be made from a metallic material selected from any type of stainless steel, silver, platinum, palladium, gold, titanium, tantalum, iridium, tungsten, cobalt, chromium, cobalt-chromium alloy 1058, cobalt-based 35N alloy, nickel-based alloy 625, a molybdenum alloy, a molybdenum alloy including about 0.4% to about 0.8% of lanthanum oxide (Li.sub.2O.sub.3), and a nickel-titanium alloy, or other suitable materials known in the art. The stents also may be made from nitinol or other shape-memory metal. Moreover, the stents may be configured in a variety of ways to provide a suitable intraluminal support structure. For example, one or more stents may be made from a woven wire structure, a laser-cut cannula, individual interconnected rings, or another pattern or design.

It can be seen from Figures 2, 3, 4 and 5 that the stents are zig-zag stents comprising struts and bends. For instance, referring to Figure 2, an intermediate stent 50 (an external stent) including struts 51, 53, 55, 57, 59 can be seen. Additional struts are also part of the stent 50 but are not visible in Figure 2. The stent 50 also has bends including bends 52, 54, 56, and 58. These struts and bends form peaks and valleys. The intermediate stents (those stents between the end stents), including stents 50 and 60, are longitudinally spaced apart such that their respective peaks are substantially aligned, as can be seen in Figures 2 and 3. For example, referring to Figure 2, it can be seen that respective peaks 62, 66 of stent 60 are aligned with respective peaks 52,56 of stent 50.

Again referring to Figure 2, it can be seen that the external wire portions are disposed within a V or an inverted V, formed between adjacent struts of one of the plurality of intermediate stents. For instance, external wire portion 113 is disposed within the inverted V formed between adjacent struts 53 and 55 of the intermediate stent 50.

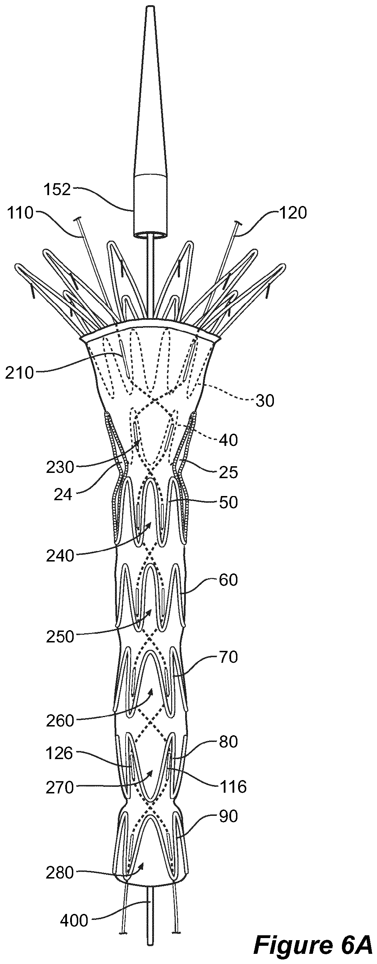

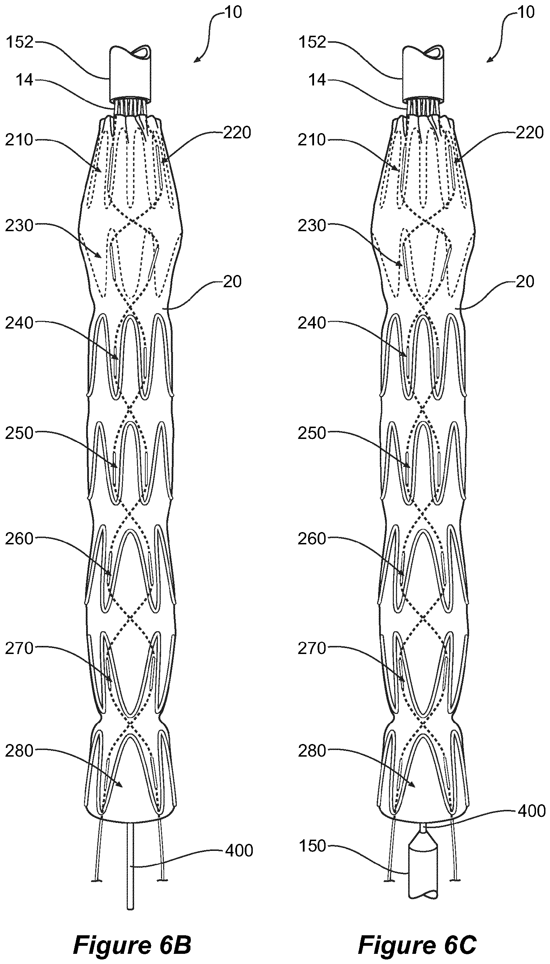

Referring now to Figure 5, the elongate receiver 300 is in the form of a temporary receiver tube having a through-bore 310. Figure 5 shows the constraint arrangement of Figures 2, 3 and 4 loaded on to a guide wire cannula 400 of a delivery device. Once in this position, the receiver tube 300 can be removed and discarded leaving the constraint arrangement shown in Figure 6A, 6B and 6C.

Embodiments of the invention may arise where no separate elongate receiver tube 300 is provided or required. With such embodiments, the constraint arrangement includes the guide wire cannula 400 of a delivery device as is shown in Figure 6A, 6B and 6C (instead of a temporary elongate receiver tube 300). However, in many applications it will be advantageous to use a disposable elongate receiver in the form of a temporary tube 300 as described above and as illustrated in Figures 2, 3 and 4.

In yet further embodiments of the invention, the temporary elongate receiving tube 300 may have a larger diameter than that shown in Figure 5. With such embodiments, the internal bore 310 of the receiver tube may be sufficiently large to allow the constraint arrangement assembly shown in Figure 2, 3 and 4 to be slid over the top of a nose cone capsule 152 of the type illustrated in Figures 6A to 6C.

In yet further embodiments of the invention, the elongate receiver may be a stylet wire or other non-hollow member. With such embodiments, the elongate receiver would simply be a place marker for insertion of a guide wire cannula 400, such as the guide wire cannula 400 illustrated in Figure is 6A, 6B and 6C.

Figures 6A to 6C show a stent graft mounted onto a deployment device with a pusher catheter 150 at one end (see Figure 6C) and a nose cone capsule 152 into which the proximally extending barbed stent 14 is received at the other end. In these drawings, no containing sheath is in place around the stent graft and so the stent graft is partially expanded under the influence of self expanding stents but complete expansion has been prevented by the wires 110 and 120.

The wires 110 and 120 can be removed by the surgeon when required. This will release the stent graft 10 from the guide wire catheter and will also allow stent graft 10 to expand under the action of the stents. This can be done when the stent graft is still mounted onto the deployment device so that the exposed stent 124 is still received in the capsule 152.

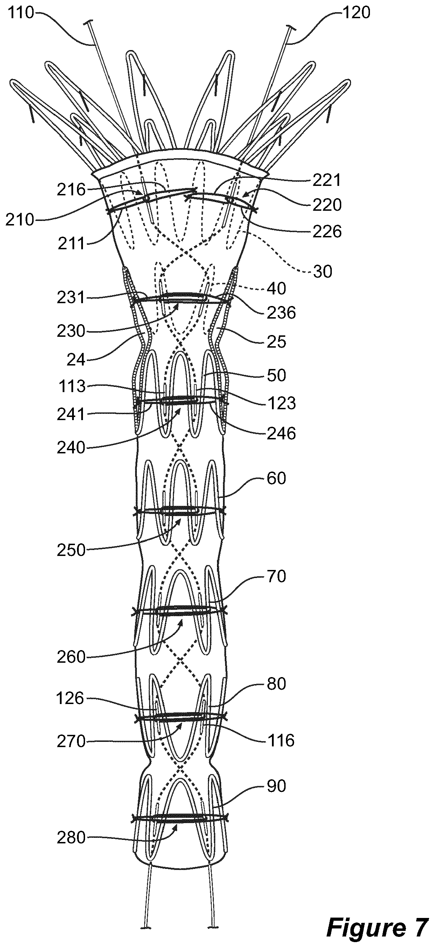

Now referring to Figure 7, an embodiment of the invention similar to that shown in Figure 3, but having diameter reducing ties or sutures is shown. A plurality of loops of thread, each loop engaged with one or other of the first and second wires and engaged around a portion of the stent graft circumferentially spaced a selected distance away from its wire, and drawn tight and tied to itself to reduce the stent graft.

The plurality of loops of thread are arranged in pairs and engage with one or other of the first and second wires 110,120 and engage around the portion of the stent graft circumferentially spaced a selected distance away from its wire. For instance, an end constraint arrangement comprising four loops of thread arranged into a first pair 210 and second pair 220 of threads (sutures) is provided, as can be seen in Figure 7. The first pair 210 is engaged with the first wire 110 and the second pair 220 is engaged with the second wire 120, as is shown in Figure 7. The first pair of threads 210 comprises a first thread 211 and a second thread 216. The second pair of threads 220 comprises a third thread 221 and a fourth thread 226.

Still referring to Figure 7, an intermediate constraint arrangement comprising a fifth and sixth loops of thread 231 and 236 arranged in a third pair 230. This intermediate constraint arrangement may be used with the other elements taught herein or separately.

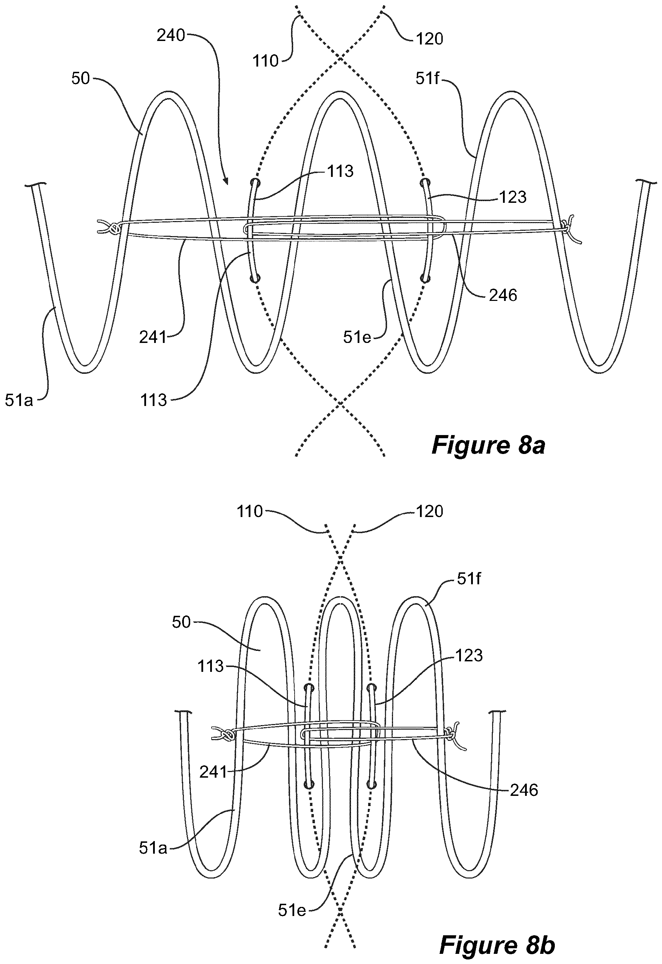

Figures 8A and 8B are detailed views showing the sutures or ties of Figure 7 in an initial expanded condition and in a constrained or reduced condition respectively. Seventh and eighth loops of thread 241 and 246 arranged in a forth pair 240 is shown in Figures 8A and 8B.

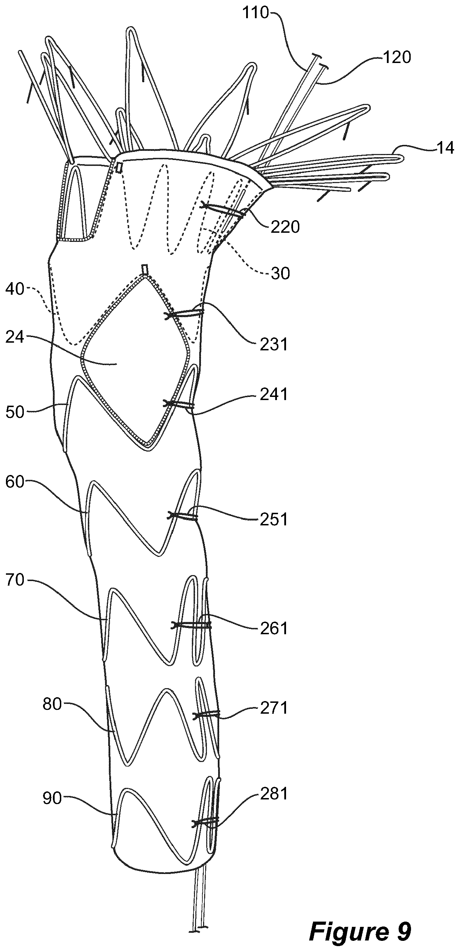

Figure 9 is an isometric side view showing the constraint arrangement of Figure 7 in a side view. This view also shows optional fenestrations 24 and 25 which can be provided for allowing access to the renal arteries as discussed previously above. This view also shows the first thread 211 of the first pair 210 of threads (sutures). It also shows the fifth, seventh, ninth, eleventh, thirteenth and fifteenth threads, 231, 241, 251, 261, 271, 281.

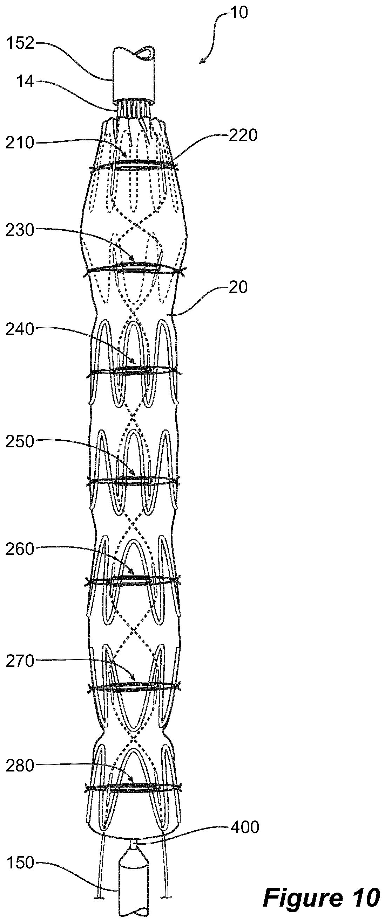

Figure 10 shows the constraint arrangement of Figure 7 as part of a delivery device. Figure 10 is similar to Fig 6C, but shows the stent graft of Figure 7 mounted onto a deployment device with a pusher catheter 150 at one end and a nose cone capsule 152 into which the proximally extending barbed stent 14 is received at the other end. In these drawings, no containing sheath is in place around the stent graft and so the stent graft is partially expanded under the influence of self-expanding stents but complete expansion has been prevented by the wires 110 and 120 and diameter reducing ties pairs 210, 220, 230, 240, 250, 260, 270 and 280.

The wires 110 and 120 can be removed by the surgeon when required. This will release the stent graft 10 from the guide wire catheter while at the same time will release the diameter reducing ties or sutures. This will also allow the stent graft 10 to expand under the action of the stents. This can be done when the stent graft is still mounted onto the deployment device so that the exposed stent 124 is still received in the capsule 152.

After removal of the wires 110 and 120, the sutures of the reducing tie pairs are released, but can remain on the outside of the stent graft. This does not cause problems as they do not interfere with blood flow and may assist with adhesion of the stent graft onto the wall of the aorta.

With the suture arrangement shown in Figures 7, 8A and 8B, labour is minimised. The arrangement also reduces the likelihood of the ties not releasing correctly due to incorrect sewing or other factors. This is important because, if fabric is caught, then this is likely to inhibit deployment of the stent graft within a lumen.

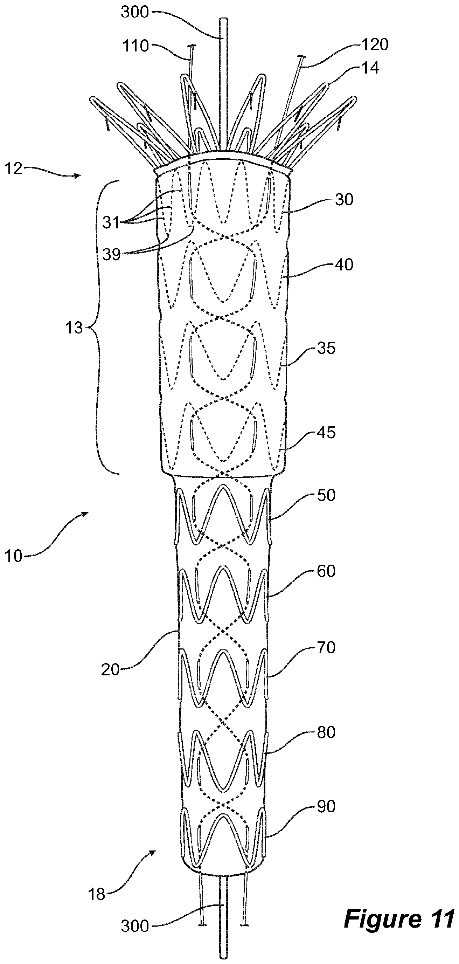

Figure 11 shows another embodiment of the invention in an isometric view. This embodiment has a longer sealing zone 13. Other embodiments of the invention, not shown, will include stent grafts of differing shapes and sizes with and without fenestrations, bifurcations and other features.

With the embodiments illustrated, the expandable external stents 50, 60, 70, 80, 90 may be of nitinol (metal alloy of nickel and titanium) whereas the internal stents 30,40 may be of stainless steel. Nitinol is super-elastic and stainless-steel is non-super elastic. In other embodiments, other suitable materials may be used.

With the embodiments illustrated, the wires 110,120 are 0.35 mm (0.014") Nitinol wires. Other wire diameters and other suitable materials may be used that are functionally similar to the wires illustrated and described above.

Throughout this specification and the claims that follow unless the context requires otherwise, the words 'comprise' and 'include' and variations such as 'comprising' and 'including' will be understood to imply the inclusion of a stated integer or group of integers but not the exclusion of any other integer or group of integers.

This application claims priority from United States patent application number US-15/347,907 and Australian patent application number 2016/256777.

## Claims

1. A temporary diameter reduction constraint arrangement (8) for a stent graft in combination with a stent graft (10), the stent graft having a proximal end (12) and a distal end (18) and comprising a biocompatible graft material tube (20) and a plurality of longitudinally spaced apart self-expanding stents (30, 40, 50, 60, 70, 80, 90) fastened thereto, including at least an end stent (30, 90) and a plurality of intermediate stents (40, 50, 60, 70, 80), the constraint arrangement comprising:
an elongate receiver (300) extending longitudinally within the graft material tube;
a first wire (110) extending longitudinally along the graft material tube in a first serpentine pattern; and
a second wire (120) extending longitudinally along the graft material tube in a second serpentine pattern,
wherein at least one of the first (110) and second (120) wires repeatedly loops over the receiver, within the graft material, along a longitudinal length of the stent graft, thereby securing the stent graft (10) to the receiver (300).

2. The constraint arrangement (8) of claim 1, wherein the first and second serpentine patterns cross each other in a criss-cross lacing pattern.

3. The constraint arrangement (8) of claim 1 or 2, wherein the first (110) and second (120) wires repeatedly cross each other within the graft material tube (20).

4. The constraint arrangement (8) of any preceding claim, wherein the first and second wires lie predominately inside the graft material tube (20).

5. The constraint arrangement (8) of any preceding claim, wherein the first (110) and second (120) wires repeatedly penetrate a tubular wall (22) of the graft material tube (20) from inside the graft material tube to outside the graft material tube and then penetrate the tubular wall (22) back from outside the graft material tube to inside the graft material tube, thereby forming a plurality of external wire portions (111, 121).

6. The constraint arrangement (8) of claim 5, wherein the external wire portions are disposed within a V, or an inverted V, formed between adjacent struts (53, 55) of one of the plurality of intermediate stents.

7. The constraint arrangement (8) of any preceding claim, wherein each intermediate stent (40, 50, 60, 70, 80) is a zig-zag stent comprising struts (51, 53, 55, 57, 59) and bends (52, 54, 56, 58) forming peaks and valleys and wherein the intermediate stents (40, 50, 60, 70, 80) are longitudinally spaced apart such that their respective peaks are substantially longitudinally aligned.

8. The constraint arrangement (8) of any preceding claim, wherein the receiver (300) is a temporary loading member, the temporary member provided to facilitate loading of the constraint arrangement onto a guide wire cannula.

9. The constraint arrangement (8) of claim 8, wherein the temporary loading member comprises a tube having a through-bore (310).

10. The constraint arrangement (8) of any preceding claim, wherein the receiver is a guide wire cannula (400).

11. The constraint arrangement (8) of any preceding claim, including a plurality of loops of thread (211, 216, 221, 226), each loop engaged with one or other of the first (110) and second (112) wires and engaged around a portion of the stent graft (10) circumferentially spaced a selected distance away from its wire, and drawn and tied to itself to reduce the stent graft.

## Patentansprüche

1. Einschränkungsbaugruppe zur vorübergehenden Durchmesserverringerung (8) für ein Stenttransplantat in Kombination mit einem Stenttransplantat (10), wobei das Stenttransplantat ein proximales Ende (12) und ein distales Ende (18) aufweist und ein Rohr (20) aus biokompatiblem Transplantatmaterial und eine Vielzahl von in Längsrichtung beabstandeten selbstexpandierenden Stents (30, 40, 50, 60, 70, 80, 90), die daran befestigt sind, umfasst, einschließlich wenigstens eines End-Stents (30, 90) und einer Vielzahl von mittleren Stents (40, 50, 60, 70, 80), wobei die Einschränkungsbaugruppe umfasst:
ein langgestrecktes Aufnahmeelement (300), das in Längsrichtung innerhalb des Rohrs aus Transplantatmaterial verläuft;
einen ersten Draht (110), der in einer ersten Serpentinenstruktur in Längsrichtung entlang des Rohrs aus Transplantatmaterial verläuft;
einen zweiten Draht (120), der in einer zweiten Serpentinenstruktur in Längsrichtung entlang des Rohrs aus Transplantatmaterial verläuft;
wobei wenigstens einer von dem ersten (110) und dem zweiten (120) Draht innerhalb des Transplantatmaterials entlang der Längsrichtung des Stenttransplantats das Aufnahmeelement wiederholt umschlingt, um das Stenttransplantat (10) an dem Aufnahmeelement (300) zu befestigen.

2. Einschränkungsbaugruppe (8) gemäß Anspruch 1, wobei sich die erste und die zweite Serpentinenstruktur in einer kreuzweisen Schnürstruktur kreuzen.

3. Einschränkungsbaugruppe (8) gemäß Anspruch 1 oder 2, wobei sich der erste (110) und der zweite (120) Draht wiederholt innerhalb des Rohrs (20) aus Transplantatmaterial kreuzen.

4. Einschränkungsbaugruppe (8) gemäß einem der vorstehenden Ansprüche, wobei der erste und der zweite Draht vorwiegend innerhalb des Rohrs (20) aus Transplantatmaterial liegen.

5. Einschränkungsbaugruppe (8) gemäß einem der vorstehenden Ansprüche, wobei der erste (110) und der zweite (120) Draht wiederholt eine Rohrwand (22) des Rohrs (20) aus Transplantatmaterial von innerhalb des Rohrs aus Transplantatmaterial nach außerhalb des Rohrs aus Transplantatmaterial durchdringen und dann die Rohrwand (22) zurück von außerhalb des Rohrs aus Transplantatmaterial nach innerhalb des Rohrs aus Transplantatmaterial durchdringen, um eine Vielzahl von außenliegenden Drahtabschnitten (111, 121) zu bilden.

6. Einschränkungsbaugruppe (8) gemäß Anspruch 5, wobei die außenliegenden Drahtabschnitte innerhalb eines V oder eines umgekehrten V angeordnet sind, das zwischen benachbarten Streben (53, 55) eines aus der Vielzahl von mittleren Stents gebildet ist.

7. Einschränkungsbaugruppe (8) gemäß einem der vorstehenden Ansprüche, wobei jeder mittlere Stent (40, 50, 60, 70, 80) ein Zickzack-Stent ist, der Streben (51, 53, 55, 57, 59) und Biegungen (52, 54, 56, 58) umfasst, die Scheitel und Täler bilden, und wobei die mittleren Stents (40, 50, 60, 70, 80) in Längsrichtung beabstandet sind, so dass ihre entsprechenden Scheitel im Wesentlichen in Längsrichtung fluchten.

8. Einschränkungsbaugruppe (8) gemäß einem der vorstehenden Ansprüche, wobei das Aufnahmeelement (300) ein zeitweiliges Ladeelement ist, wobei das zeitweilige Element bereitgestellt ist, um das Laden der Einschränkungsbaugruppe auf eine Führungsdrahtkanüle zu erleichtern.

9. Einschränkungsbaugruppe (8) gemäß Anspruch 8, wobei das zeitweilige Ladeelement ein Rohr mit einer Durchgangsbohrung (310) umfasst.

10. Einschränkungsbaugruppe (8) gemäß einem der vorstehenden Ansprüche, wobei das Aufnahmeelement eine Führungsdrahtkanüle (400) ist.

11. Einschränkungsbaugruppe (8) gemäß einem der vorstehenden Ansprüche, umfassend eine Vielzahl von Fadenschlaufen (211, 216, 221, 226), wobei jede Schlaufe mit einem oder dem anderen von dem ersten (110) und dem zweiten (112) Draht in Eingriff steht und in einem ausgewählten Abstand von seinem Draht entfernt umlaufend beabstandet um einen Teil des Stenttransplantats (10) in Eingriff steht und an sich selbst gezogen und gebunden ist, um das Stenttransplantat zu verkleinern.

## Revendications

1. Agencement de contrainte de réduction de diamètre temporaire (8) pour un greffon de stent en association avec un greffon de stent (10), le greffon de stent présentant une extrémité proximale (12) et une extrémité distale (18) et comprenant un tube en matériau de greffon biocompatible (20) et une pluralité de stents autodéployables espacés longitudinalement (30, 40, 50, 60, 70, 80, 90) fixés à celui-ci, comprenant au moins un stent d'extrémité (30, 90) et une pluralité de stents intermédiaires (40, 50, 60, 70, 80), l'agencement de contrainte comprenant :
un récepteur allongé (300) s'étendant longitudinalement à l'intérieur du tube de matériau de greffon ;
un premier fil (110) s'étendant longitudinalement le long du tube de matériau de greffon dans un premier motif en serpentin ; et
un second fil (120) s'étendant longitudinalement le long du tube de matériau de greffon dans un second motif en serpentin,
dans lequel au moins un des premier (110) et second (120) fils s'enroule de manière répétée sur le récepteur, à l'intérieur du matériau de greffon, sur une longueur longitudinale du greffon de stent, ce qui permet de fixer le greffon de stent (10) au récepteur (300).

2. Agencement de contrainte (8) selon la revendication 1, dans lequel les premier et second motifs en serpentin se croisent l'un l'autre dans un motif d'entrelacement.

3. Agencement de contrainte (8) selon la revendication 1 ou 2, dans lequel les premier (110) et second (120) fils se croisent l'un l'autre de manière répétée à l'intérieur du tube de matériau de greffon (20).

4. Agencement de contrainte (8) selon l'une quelconque des revendications précédentes, dans lequel les premier et second fils se trouvent majoritairement à l'intérieur du tube de matériau de greffon (20).

5. Agencement de contrainte (8) selon l'une quelconque des revendications précédentes, dans lequel les premier (110) et second (120) fils pénètrent de manière répétée dans une paroi tubulaire (22) du tube de matériau de greffon (20) depuis l'intérieur du tube de matériau de greffon vers l'extérieur du tube de matériau de greffon puis pénètrent à nouveau dans la paroi tubulaire (22) depuis l'extérieur du tube de matériau de greffon vers l'intérieur du tube de matériau de greffon, ce qui permet de former une pluralité de parties fil externe (111, 121).

6. Agencement de contrainte (8) selon la revendication 5, dans lequel les parties fil externe sont disposées dans un V, ou un V inversé, formé entre des entretoises adjacentes (53, 55) de l'un des stents de la pluralité de stents intermédiaires.

7. Agencement de contrainte (8) selon l'une quelconque des revendications précédentes, dans lequel chaque stent intermédiaire (40, 50, 60, 70, 80) est un stent en zigzag comprenant des entretoises (51, 53, 55, 57, 59) et des coudes (52, 54, 56, 58) formant des crêtes et des vallées et dans lequel les stents intermédiaires (40, 50, 60, 70, 80) sont espacés longitudinalement de sorte que leurs crêtes respectives soient sensiblement alignées longitudinalement.

8. Agencement de contrainte (8) selon l'une quelconque des revendications précédentes, dans lequel le récepteur (300) est un élément de chargement temporaire, l'élément temporaire étant prévu pour faciliter le chargement de l'agencement de contrainte sur une canule de fil guide.

9. Agencement de contrainte (8) selon la revendication 8, dans lequel l'élément de chargement temporaire comprend un tube possédant un alésage traversant (310).

10. Agencement de contrainte (8) selon l'une quelconque des revendications précédentes, dans lequel le récepteur est une canule (400) de fil guide.

11. Agencement de contrainte (8) selon l'une quelconque des revendications précédentes, comprenant une pluralité de boucles de fil (211, 216, 221, 226), chaque boucle étant en prise avec un ou l'autre des premier (110) et second (112) fils et en prise autour d'une partie du greffon de stent (10) circonférentiellement espacé de son fil par une distance sélectionnée, et tirée et fixée sur elle-même pour réduire le greffon de stent.
